(19) Europäisches Patentamt
European Patent Office
Office européen des brevets

(11) EP 3 470 107 A1

(12) EUROPEAN PATENT APPLICATION

(43) Date of publication:
17.04.2019 Bulletin 2019/16

(51) Int Cl.:
A61M 27/00 (2006.01)     A61M 1/00 (2006.01)
A61F 13/20 (2006.01)     A61M 25/04 (2006.01)
A61M 25/10 (2013.01)     A61F 13/15 (2006.01)

(21) Application number: 18183523.2

(22) Date of filing: 20.07.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 20.07.2015   US 201562194585 P
30.11.2015   US 201562260966 P
14.01.2016   US 201662278721 P
25.02.2016   US 201662300025 P

(62) Document number(s) of the earlier application(s) in
accordance with Art. 76 EPC:
16828454.5 / 3 325 076

(27) Previously filed application:
20.07.2016 PCT/US2016/043101

(71) Applicant: Strataca Systems Limited
Floriana, FRN 1504 (MT)

(72) Inventors:
• ERBEY, John, R., II
Milton, GA 30004 (US)
• UPPERCO, Jacob, L.
Atlanta, GA 30308 (US)
• FISHER, Michael, Allen
Lawrenceville, GA 30045 (US)
• STRANE, Patrick, William
Atlanta, GA 30309 (US)
• BLACK, Lance, Michael
Pearland, TX 77584 (US)

(74) Representative: Rentsch Partner AG
Bellerivestrasse 203
Postfach
8034 Zürich (CH)

Remarks:
This application was filed on 13-07-2018 as a
divisional application to the application mentioned
under INID code 62.

(54) URETERAL AND BLADDER CATHETERS

(57) A bladder catheter comprising a proximal portion and a distal portion. The distal portion comprises an inwardly facing side (240), which comprises one or more perforations (230). The distal portion further comprises an outwardly facing side (242). When negative pressure is applied through the bladder catheter, fluid is drawn into the bladder catheter through the one or more perforations while bladder walls are prevented from appreciably occluding the one or more perforations.

FIG. 5

EP 3 470 107 A1

**Description**

**CROSS REFERENCE TO RELATED APPLICATIONS**

This application claims priority to United States Provisional Application No. 62/194,585, filed July 20, 2015, United States Provisional Application No. 62/260,966, filed November 30, 2015, United States Provisional Application No. 62/278,721, filed January 14, 2016, and United States Provisional Application No. 62/300,025 filed February 25, 2016, each of which is incorporated herein by reference in its entirety.

**BACKGROUND**

Technical Field

The present disclosure relates to devices and methods for treating impaired renal function across a variety of disease states and, in particular, to devices and methods for collection of urine and inducement of negative pressure in the bladder.

Background

The renal or urinary system includes a pair of kidneys, each kidney being connected by a ureter to the bladder, and a urethra for draining urine produced by the kidneys from the bladder. The kidneys perform several vital functions for the human body including, for example, filtering the blood to eliminate waste in the form of urine. The kidneys also regulate electrolytes (e.g., sodium, potassium and calcium) and metabolites, blood volume, blood pressure, blood pH, fluid volume, production of red blood cells, and bone metabolism. Adequate understanding of the anatomy and physiology of the kidneys is useful for understanding the impact that altered hemodynamics other fluid overload conditions have on their function.

In normal anatomy, the two kidneys are located retroperitoneally in the abdominal cavity. The kidneys are bean-shaped encapsulated organs. Urine is formed by nephrons, the functional unit of the kidney, and then flows through a system of converging tubules called collecting ducts. The collecting ducts join together to form minor calyces, then major calyces, which ultimately join near the concave portion of the kidney (renal pelvis). A major function of the renal pelvis is to direct urine flow to the ureter. Urine flows from the renal pelvis into the ureter, a tube-like structure that carries the urine from the kidneys into the bladder. The outer layer of the kidney is called the cortex, and is a rigid fibrous encapsulation. The interior of the kidney is called the medulla. The medulla structures are arranged in pyramids.

Each kidney is made up of approximately one million nephrons. Each nephron includes the glomerulus, Bowman's capsule, and tubules. The tubules include the proximal convoluted tubule, the loop of Henle, the distal convoluted tubule, and the collecting duct. The nephrons contained in the cortex layer of the kidney are distinct from the anatomy of those contained in the medulla. The principal difference is the length of the loop of Henle. Medullary nephrons contain a longer loop of Henle, which, under normal circumstances, allows greater regulation of water and sodium reabsorption than in the cortex nephrons.

The glomerulus is the beginning of the nephron, and is responsible for the initial filtration of blood. Afferent arterioles pass blood into the glomerular capillaries, where hydrostatic pressure pushes water and solutes into Bowman's capsule. Net filtration pressure is expressed as the hydrostatic pressure in the afferent arteriole minus the hydrostatic pressure in Bowman's space minus the osmotic pressure in the efferent arteriole.

$$Net\ Filtration\ Pressure = Hydrostatic\ Pressure\ (Afferent$$
$$Arteriole) - Hydrostatic\ Pressure\ (Bowman's\ Space) - Osmotic$$
$$Pressure\ (Efferent\ Arteriole)\ (Equation\ 1)$$

The magnitude of this net filtration pressure defined by Equation 1 determines how much ultra-filtrate is formed in Bowman's space and delivered to the tubules. The remaining blood exits the glomerulus via the efferent arteriole. Normal glomerular filtration, or delivery of ultra-filtrate into the tubules, is about 90 ml/min/1.73m$^2$.

The glomerulus has a three-layer filtration structure, which includes the vascular endothelium, a glomerular basement membrane, and podocytes. Normally, large proteins such as albumin and red blood cells, are not filtered into Bowman's space. However, elevated glomerular pressures and mesangial expansion create surface area changes on the basement membrane and larger fenestrations between the podocytes allowing larger proteins to pass into Bowman's space.

Ultra-filtrate collected in Bowman's space is delivered first to the proximal convoluted tubule. Re-absorption and secretion of water and solutes in the tubules is performed by a mix of active transport channels and passive pressure gradients. The proximal convoluted tubules normally reabsorb a majority of the sodium chloride and water, and nearly all glucose and amino acids that were filtered by the glomerulus. The loop of Henle has two components that are designed to concentrate wastes in the urine. The descending limb is highly water permeable and reabsorbs most of the remaining water. The ascending limb reabsorbs 25% of the remaining sodium chloride, creating a concentrated urine, for example, in terms of urea and creatinine. The distal convoluted tubule normally reabsorbs a small proportion of sodium chloride, and the osmotic gradient creates conditions for the water to follow.

Under normal conditions, there is a net filtration of approximately 14 mmHg. The impact of venous congestion can be a significant decrease in net filtration, down to approximately 4 mmHg. *See* Jessup M., The cardiorenal syndrome: Do we need a change of strategy or a change of tactics?, JACC 53(7):597-600, 2009 (hereinafter "Jessup"). The second filtration stage occurs at the proximal tubules. Most of the secretion and absorption from urine occurs in tubules in the medullary nephrons. Active transport of sodium from the tubule into the interstitial space initiates this process. However, the hydrostatic forces dominate the net exchange of solutes and water. Under normal circumstances, it is believed that 75% of the sodium is reabsorbed back into lymphatic or venous circulation. However, because the kidney is encapsulated, it is sensitive to changes in hydrostatic pressures from both venous and lymphatic congestion. During venous congestion the retention of sodium and water can exceed 85%, further perpetuating the renal congestion. *See* Verbrugge et al., The kidney in congestive heart failure: Are natriuresis, sodium, and diruetucs really the good, the bad and the ugly? European Journal of Heart Failure 2014:16,133-42 (hereinafter "Verbrugge").

Venous congestion can lead to a prerenal form of acute kidney injury (AKI). Prerenal AKI is due to a loss of perfusion (or loss of blood flow) through the kidney. Many clinicians focus on the lack of flow into the kidney due to shock. However, there is also evidence that a lack of blood flow out of the organ due to venous congestion can be a clinically important sustaining injury. *See* Damman K, Importance of venous congestion for worsening renal function in advanced decompensated heart failure, JACC 17:589-96, 2009 (hereinafter "Damman").

Prerenal AKI occurs across a wide variety of diagnoses requiring critical care admissions. The most prominent admissions are for sepsis and Acute Decompensated Heart Failure (ADHF). Additional admissions include cardiovascular surgery, general surgery, cirrhosis, trauma, burns, and pancreatitis. While there is wide clinical variability in the presentation of these disease states, a common denominator is an elevated central venous pressure. In the case of ADHF, the elevated central venous pressure caused by heart failure leads to pulmonary edema, and, subsequently, dyspnea in turn precipitating the admission. In the case of sepsis, the elevated central venous pressure is largely a result of aggressive fluid resuscitation. Whether the primary insult was low perfusion due to hypovolemia or sodium and fluid retention, the sustaining injury is the venous congestion resulting in inadequate perfusion.

Hypertension is another widely recognized state that creates perturbations within the active and passive transport systems of the kidney(s). Hypertension directly impacts afferent arteriole pressure and results in a proportional increase in net filtration pressure within the glomerulus. The increased filtration fraction also elevates the peritubular capillary pressure, which stimulates sodium and water re-absorption. *See* Verbrugge.

Because the kidney is an encapsulated organ, it is sensitive to pressure changes in the medullary pyramids. The elevated renal venous pressure creates congestion that leads to a rise in the interstitial pressures. The elevated interstitial pressures exert forces upon both the glomerulus and tubules. *See* Verburgge. In the glomerulus, the elevated interstitial pressures directly oppose filtration. The increased pressures increase the interstitial fluid, thereby increasing the hydrostatic pressures in the interstitial fluid and peritubular capillaries in the medulla of the kidney. In both instances, hypoxia can ensue leading to cellular injury and further loss of perfusion. The net result is a further exacerbation of the sodium and water re-absorption creating a negative feedback. *See* Verbrugge, 133-42. Fluid overload, particularly in the abdominal cavity is associated with many diseases and conditions, including elevated intra-abdominal pressure, abdominal compartment syndrome, and acute renal failure. Fluid overload can be addressed through renal replacement therapy. *See* Peters, C.D., Short and Long-Term Effects of the Angiotensin II Receptor Blocker Irbesartanon Intradialytic Central Hemodynamics: A Randomized Double-Blind Placebo-Controlled One-Year Intervention Trial (the SAFIR Study), PLoS ONE (2015) 10(6): e0126882. doi:10.1371/journal.pone.0126882 (hereinafter "Peters"). However, such a clinical strategy provides no improvement in renal function for patients with the cardiorenal syndrome. *See* Bart B, Ultrafiltration in decompensated heart failure with cardiorenal syndrome, NEJM 2012;367:2296-2304 (hereinafter "Bart").

In view of such problematic effects of fluid retention, devices and methods for improving removal of urine from the urinary tract and, specifically for increasing quantity and quality of urine output from the kidneys, are needed.

## SUMMARY

The present disclosure improves upon previous systems by providing a specialized (non-Foley) catheter for deployment within the bladder. The catheter of the present disclosure comprises an indwelling portion to be positioned within the bladder having restraints or anchors for passive fixation with superior and/or inferior portions of the bladder wall. A proximal restraint or anchor, within the bladder, can be designed to seal the urethra from air and fluid leaks. A distal anchor within the bladder can be designed to restrain the superior bladder wall as it collapses, allowing obstruction free delivery of negative pressure, collection of the urine produced, and avoidance of mucosal trauma. Further, a urine collection system comprising the catheter includes sensing devices for monitoring urine flow and for using urine flow and conductivity assessment to guide the negative pressure delivered by the system to optimize sodium and water excretion.

Non-limiting examples, aspects, or embodiments of the present invention will now be described in the following numbered clauses:

Clause 1: A urine collection catheter configured to be deployed in a patient's bladder, the catheter comprising: a conduit comprising a proximal end and an open distal end; a drainage tube positioned at least partially within the conduit, the drainage tube comprising a proximal end, a distal end, and one or more fluid ports or perforations for permitting fluid flow into a drainage lumen defined by the drainage tube; and a bladder superior wall support comprising a support cap and a plurality of support members extending from a proximal surface of the support cap through the open distal end of the conduit, and being capable of being moved between a retracted position and a deployed position, wherein, in the deployed position, the distal end of the drainage tube is spaced apart from the support cap, such that the support cap supports portions of the superior wall of the bladder from occluding the one or more fluid ports or perforations of the drainage tube.

Clause 2: The catheter of clause 1, wherein the support cap is configured to inhibit the superior bladder wall from occluding the one or more ports or perforations upon delivery of negative pressure to the bladder and/or kidneys through the drainage tube.

Clause 3: The catheter of clause 1 or clause 2, wherein the support cap is configured to inhibit the superior bladder wall from contacting ureteral orifices of the bladder upon delivery of negative pressure to the bladder and/or kidneys through the drainage lumen.

Clause 4: The catheter of any of clauses 1 to 3, wherein the support members comprise flexible tines, and wherein the support cap comprises a flexible cover mounted to and supported by the plurality of tines.

Clause 5: The catheter of clause 4, wherein the flexible tines comprise a shape-memory alloy configured to move to the deployed position at a temperature above ambient room temperature.

Clause 6: The catheter of clause 5, wherein the flexible cover is formed from a material that does not appreciably abrade, irritate, or damage a mucosal lining of the bladder walls or of a urethra when positioned adjacent to the mucosal lining of the bladder walls or the urethra.

Clause 7: The catheter of any of clauses 1 to 6, wherein the bladder superior wall support is configured to maintain its form when in contact with the superior wall of the bladder.

Clause 8: The catheter of any of clauses 1 to 3, wherein the support cap comprises an inflatable balloon.

Clause 9: The catheter of clause 8, further comprising an inflation lumen at least partially disposed within the conduit and configured to conduct a fluid or gas into an interior of the balloon for inflation of the balloon.

Clause 10: A urine collection catheter configured to be deployed in a patient's bladder, the catheter comprising: at least one tubular body comprising a proximal end, a distal end, a sidewall extending therebetween, and one or more fluid ports or perforations for permitting fluid flow into a drainage lumen defined by the tubular body; and a bladder superior wall support comprising a support cap connected to and extending radially from a portion of the distal end of the at least one tubular body, the support cap comprising a curved distal surface, the support cap being capable of being moved between a retracted position and a deployed position to support a superior wall of the bladder thereby inhibiting the superior bladder wall from occluding the one or more fluid ports or perforations.

Clause 11: The catheter of clause 10, wherein the bladder superior wall support is configured to inhibit the superior bladder wall from occluding the one or more ports or perforations upon delivery of negative pressure to the bladder and/or kidneys through the drainage lumen.

Clause 12: The catheter of clause 10, wherein the bladder superior wall support is configured to inhibit the superior bladder wall from contacting ureteral orifices of the bladder upon delivery of negative pressure to the bladder and/or kidneys through the drainage lumen.

Clause 13: The catheter of any of clauses 10 to 13, wherein the bladder superior wall support comprises a plurality of support members extending radially from the tubular body, and wherein the support cap comprises a flexible cover mounted to and supported by the plurality of support members.

Clause 14: The catheter of clause 13, wherein the support members comprise flexible tines formed from a shape-memory alloy, the flexible tines being configured to extend to the deployed position at a temperature above ambient room temperature.

Clause 15: The catheter of clause 13 or clause 14, wherein the flexible cover is formed from a material that does not appreciably abrade, irritate, or damage a mucosal lining of the bladder walls or of a urethra when positioned adjacent to the mucosal lining of the bladder walls or the urethra.

Clause 16: The catheter of any of clauses 10 to 15, wherein the bladder superior wall support comprises an inflatable balloon.

Clause 17: The catheter of clause 16, further comprising an inflation lumen configured to conduct a fluid or gas into an interior of the balloon for inflation of the balloon.

Clause 18: The catheter of clause 16 or clause 17, wherein the inflatable balloon comprises a bulbous portion and a plurality of lobes extending proximally therefrom, and wherein the one or more ports or perforations are positioned between adjacent lobes.

Clause 19: The catheter of any of clauses 10 to 18, further comprising a filter positioned over the one or more ports or perforations.

Clause 20: The catheter of any of clauses 10 to 19, further comprising an absorbent sponge positioned over the one or more ports or perforations.

Clause 21: The catheter of any of clauses 10 to 20, further comprising a bladder inferior wall support configured to contact an inferior wall of the bladder.

Clause 22: The catheter of clause 21, wherein the bladder inferior wall support comprises one or more support members extending radially from the tubular body and a cover to the one or more support members.

Clause 23: The catheter of clause 22, wherein the cover of the bladder inferior wall support comprises a material that does not appreciably abrade, irritate, or damage a mucosal lining of the bladder walls or of a urethra when positioned adjacent to the mucosal lining of the bladder walls or the urethra.

Clause 24: A system for drawing urine from the bladder of a patient, the system comprising: a urine collection catheter comprising: at least one tubular body comprising a proximal end, a distal end, a sidewall extending therebetween, and one or more fluid ports or perforations for permitting fluid flow into a drainage lumen defined by the tubular body; and a bladder superior wall support comprising a support cap defining a curved distal surface connected to and extending radially from a portion of the distal end of the at least one tubular body, the support cap being capable of being moved between a retracted position and a deployed position to support a superior wall of the bladder to inhibit portions of the superior wall of the bladder from occluding the one or more fluid ports or perforations; and a pump in fluid connection with the drainage lumen of the tubular body, wherein the pump is configured to introduce negative pressure through the tubular body to the bladder to draw urine from the bladder.

Clause 25: The system of clause 24, further comprising one or more sensors in fluid communication with the drainage lumen of the tubular body for measuring information representative of a physiological condition of the patient.

Clause 26: The system of clause 25, wherein the one or more sensors are configured to measure one or more of capacitance, analyte concentration, and temperature of urine within the tubular body.

Clause 27: The system of clause 25 or clause 26, wherein the pump comprises: a processor comprising computer readable memory including programming instructions that, when executed, cause the processor to: receive the information from the one or more sensors, and adjust an operating parameter of the pump based, at least in part, on the information received from the one or more sensors to increase or decrease the negative pressure in the tubular body to adjust flow of urine therethrough.

Clause 28: The system of clause 27, wherein the pump further comprises a data transmitter in communication with the processor, the data transmitter being configured to provide the information from the one or more sensors to an external source.

Clause 29: The system of any of clauses 24 to 28, wherein the pump is capable of continuous operation for between 8 and 24 hours.

Clause 30: The system of any of clauses 24 to 29, wherein the pump provides a sensitivity of 10 mmHg or less.

Clause 31: The system of any of clauses 24 to 30, wherein the pump is configured to provide intermittent negative pressure.

Clause 32: The system of any of clauses 24 to 31, wherein the pump is configured to alternate between providing negative pressure and providing positive pressure.

Clause 33: The system of any of clauses 24 to 31, wherein the pump is configured to alternate between providing negative pressure and equalizing pressure to atmosphere.

Clause 34: A urine collection catheter configured to be deployed within a patient's bladder, the catheter comprising: a tubular body comprising a proximal portion configured to be positioned in at least a portion of a patient's urethra and a distal portion configured to be positioned in a patient's bladder, the distal portion comprising a coiled retention portion, wherein the retention portion comprises at least a first coil having a first diameter, a second coil having a second diameter, the first diameter being less than the second diameter, and a plurality of perforations disposed on a radially inwardly facing side of a sidewall of the retention portion.

Clause 35: The catheter of clause 34, wherein the first coil is proximal to the second coil.

Clause 36: The catheter of clause 34 or clause 35, wherein, prior to insertion into a patient's urinary tract, a portion of the tubular body that is proximal to the retention portion defines a straight or curvilinear central axis, and wherein the first coil and the second coil of the retention portion extend about an axis that is at least partially coextensive with the straight or curvilinear central axis of the portion of the drainage lumen.

Clause 37: The catheter of clause 36, wherein, in the retention portion, a total surface area for the perforations on the radially inwardly facing side of the sidewall of the tubular body is greater than a total surface area of perforations on the radially outwardly facing side of the sidewall of the tubular body.

Clause 38: The catheter of any of clauses 34 to 38, wherein, in the retention portion, a radially outwardly facing side of the sidewall of the tubular body is free from perforations.

Clause 39: A urine catheter comprising: a tubular body comprising a proximal end, a distal end, and a sidewall extending therebetween; and an indwelling portion adjacent to the distal end of the tubular body, the indwelling portion comprising a first surface configured to support a superior wall of a bladder, a second surface configured to contact an inferior wall of the bladder, and a linear portion of the sidewall extending between the first surface and the second surface, wherein the first surface and the second surface each comprise a flexible material, and wherein the first surface and the second surface are supported by a plurality of support members.

Clause 40: The catheter of clause 39, wherein the flexible material does not appreciably abrade, irritate, or damage a mucosal lining of the bladder walls or of a urethra when positioned adjacent to the mucosal lining of the bladder walls or the urethra.

Clause 41: The catheter of clause 39 or clause 40, wherein the indwelling portion insulates the superior wall of the bladder from a trigone region of the bladder.

Clause 42: The catheter of any of clauses 39 to 41, wherein the first surface and the second surface of the indwelling portion are transitionable between a contracted position and a deployed position.

Clause 43: The catheter of clause 42, wherein, in the deployed position, the first surface and the second surface maintain their form when in contact with the respective superior and inferior walls of the bladder.

Clause 44: The catheter of clause 42 or clause 43, wherein the flexible material contracts when the first surface and the second surface are in the contracted position and expands when the first surface and the second surface are in the deployed position.

Clause 45: The catheter of any of clauses 42 to 44, wherein the second surface of the indwelling portion provides a seal for an opening of a urethra of the bladder when in the deployed position.

Clause 46: The catheter of any of clauses 42 to 45, wherein the first surface of the indwelling portion contacts the superior wall of the bladder when in the deployed position so as to avoid obstruction of one or more ureter openings of the bladder.

Clause 47: The catheter of any of clauses 42 to 46, wherein a section of the indwelling portion between the first surface and the second surface is not covered with the flexible material at least when the first surface and the second surface are in the deployed position.

Clause 48: The catheter of any of clauses 42 to 47, further comprising a release mechanism configured to activate the first surface and the second surface from the contracted position to the deployed position.

Clause 49: The catheter of any of clauses 39 to 48, wherein the flexible material covers at least a portion of the plurality of support members.

Clause 50: The catheter of any of clauses 39 to 49, wherein the plurality of support members is drawn against the tube in the contracted position, and the plurality of support members extends outward from the tube in the deployed position.

Clause 51: The catheter of any of clauses 39 to 50, wherein the plurality of support members are formed of a shape-memory alloy.

Clause 52: The catheter of any of clauses 39 to 51, wherein the linear portion of the sidewall extending between the first surface and the second surface comprises one or more perforations extending through the sidewall to permit fluid flow therethrough into a fluid receiving portion of the tube.

Clause 53: A method of inducing a negative pressure to a bladder of a patient for enhancing urine excretion therefrom, the method comprising: inserting a distal portion of a tubular body of a urine collection catheter into the patient's bladder; deploying a support cap connected to and extending radially from a portion of the distal end of the tubular body, such that the support cap is in contact with the bladder superior wall; and inducing a negative pressure through a drainage lumen of the tubular body to draw urine from the bladder into the drainage lumen.

Clause 54: The method of clause 53, further comprising positioning a bladder inferior wall support in contact with an inferior wall of the patient's bladder.

Clause 55: The method of clause 54, wherein positioning the bladder inferior wall support comprises positioning the support over an opening of a urethra to seal the bladder.

Clause 56: The method of clause 54 or clause 55, wherein the bladder inferior wall support is separate from the support cap and is supported by a plurality of support members extending radially from the tubular body.

Clause 57: The method of any of clauses 53 to 56, wherein deploying the support cap comprises preventing the distal surface structure from occluding ureteral openings of the bladder.

Clause 58: The method of any of clauses 53 to 57, wherein inducing the negative pressure in the tubular body comprises coupling a mechanical pump in fluid communication with a proximal end of the tubular body to draw urine from the bladder into the tubular body.

BRIEF DESCRIPTION OF THE DRAWINGS

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limit of the invention.

Further features and other examples and advantages will become apparent from the following detailed description made with reference to the drawings in which:

FIG. 1 is a schematic drawing of a urine collection catheter device deployed within the bladder of a male patient according to an example of the disclosure;

FIG. 2 is a schematic drawing of another exemplary urine collection catheter device deployed within a patient's bladder according to an example of the disclosure;

FIG. 3A is a perspective view of an indwelling retention portion the urine collection catheter device of FIG. 1;

FIG. 3B is a cross-sectional view taken along line B-B of the indwelling retention portion of the urine collection catheter device of FIG. 3A;

FIG. 3C is a cross-sectional view taken along line C-C of the indwelling retention portion of FIG. 3A;

FIG. 4 is a perspective view of an indwelling retention portion of a urine collection catheter according to another example of the disclosure;

FIG. 5 is a perspective view of an indwelling retention portion of a urine collection catheter according to another example of the disclosure;

FIG. 6 is a perspective view of an indwelling retention portion of a urine collection catheter according to another example of the disclosure;

FIG. 7A is a schematic drawing of an indwelling retention portion of the catheter device of FIG. 2 in a contracted state;

FIG. 7B is a schematic drawing of the indwelling portion of the catheter device of FIG. 2 in a deployed state;

FIG. 8A is a schematic drawing of an indwelling retention portion of another exemplary urine collection catheter device in a contracted state, according to an embodiment of the disclosure;

FIG. 8B is a schematic drawing of the indwelling retention portion of the catheter device of FIG. 8A in a deployed state;

FIG. 9A is a perspective view of an indwelling retention portion of another exemplary urine collection catheter device according to an example of the disclosure;

FIG. 9B is a partial cross-sectional view of a portion of the indwelling retention portion of the catheter device of FIG. 9A;

FIG. 10A is a perspective view of an indwelling retention portion of another exemplary urine collection catheter device according to an example of the disclosure;

FIG. 10B is a partial cross-sectional view of a portion of the urine collection catheter of FIG. 10A;

FIG. 11A is a perspective view of an indwelling portion of another exemplary urine collection catheter according to an example of the disclosure;

FIG. 11B is a cross-sectional view of the urine collection catheter of FIG. 11A;

FIG. 12 is a front view of an indwelling retention portion of another exemplary urine collection device according to an example of the disclosure;

FIG. 13 is a schematic drawing of a urine collection device including both an indwelling portion and an external portion according to an example of the disclosure;

FIG. 14 is a system for inducing a negative pressure in the bladder of a patient including a urine collection catheter device configured to be deployed in a patient's bladder according to an example of the disclosure;

FIGS. 15A and 15B are schematic drawings of a pump for use with the system of FIG. 14; and

FIG. 16 is a flow chart of a process for inducing a negative pressure in a patient's bladder according to an example of the disclosure.

DETAILED DESCRIPTION OF THE INVENTION

As used herein, the singular form of "a", "an", and "the" include plural referents unless the context clearly dictates otherwise.

As used herein, the terms "right", "left", "top", and derivatives thereof shall relate to the invention as it is oriented in the drawing figures. The term "proximal" refers to the portion of the catheter device that is manipulated or contacted by a user. The term "distal" refers to the opposite end of the catheter device that is configured to be inserted into a patient. However, it is to be understood that the invention can assume various alternative orientations and, accordingly, such terms are not to be considered as limiting. Also, it is to be understood that the invention can assume various alternative variations and stage sequences, except where expressly specified to the contrary. It is also to be understood that the specific devices and processes illustrated in the attached drawings, and described in the following specification, are examples. Hence, specific dimensions and other physical characteristics related to the embodiments disclosed herein are not to be considered as limiting.

Unless indicated to the contrary, the numerical parameters set forth in the following specification and attached claims are approximations that can vary depending upon the desired properties sought to be obtained by the present disclosure.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

Also, it should be understood that any numerical range recited herein is intended to include all sub-ranges subsumed therein. For example, a range of "1 to 10" is intended to include any and all sub-ranges between and including the recited minimum value of 1 and the recited maximum value of 10, that is, all sub-ranges beginning with a minimum value equal to or greater than 1 and ending with a maximum value equal to or less than 10, and all sub-ranges in-between, e.g., 1 to 6.3, or 5.5 to 10, or 2.7 to 6.1.

As used herein, the terms "communication" and "communicate" refer to the receipt or transfer of one or more signals, messages, commands, or other type of data. For one unit or component to be in communication with another unit or component means that the one unit or component is able to directly or indirectly receive data from and/or transmit data to the other unit or component. This can refer to a direct or indirect connection that can be wired and/or wireless in nature. Additionally, two units or components can be in communication with each other even though the data transmitted can be modified, processed, routed, and the like, between the first and second unit or component. For example, a first unit can be in communication with a second unit even though the first unit passively receives data and does not actively transmit data to the second unit. As another example, a first unit can be in communication with a second unit if an intermediary unit processes data from one unit and transmits processed data to the second unit. It will be appreciated that numerous other arrangements are possible.

Fluid retention and venous congestion are central problems in the progression to advanced renal disease. Excess sodium ingestion coupled with relative decreases in excretion leads to isotonic volume expansion and secondary compartment involvement. In some examples, the present invention is generally directed to devices and methods for facilitating drainage of urine or waste from the bladder, ureter, and/or kidney(s) of a patient. In some examples, the present invention is generally directed to devices and methods for inducing a negative pressure in the bladder of a patient. While not intending to be bound by any theory, it is believed that applying a negative pressure to the bladder can offset the medullary nephron tubule re-absorption of sodium and water in some situations. Offsetting re-absorption of sodium and water can increase urine production, decrease total body sodium, and improve erythrocyte production. Since the intra-medullary pressures are driven by sodium and, therefore, volume overload, the targeted removal of excess sodium enables maintenance of volume loss. Removal of volume restores medullary hemostasis. Normal urine production is 1.48-1.96 L/day (or 1-1.4 ml/min).

Fluid retention and venous congestion are also central problems in the progression of prerenal AKI. Specifically, AKI can be related to loss of perfusion or blood flow through the kidney(s). Accordingly, in some examples, the present invention facilitates improved renal hemodynamics and increases urine output for the purpose of relieving or reducing venous congestion. Further, it is anticipated that treatment and/or inhibition of AKI positively impacts and/or reduces the occurrence of other conditions, for example, reduction or inhibition of worsening renal function in patients with NYHA Class III and/or Class IV heart failure. Classification of different levels of heart failure are described in The Criteria Committee of the New York Heart Association, (1994), Nomenclature and Criteria for Diagnosis of Diseases of the Heart and Great Vessels, (9th ed.), Boston: Little, Brown & Co. pp. 253-256, the disclosure of which is incorporated by reference herein in its entirety. Reduction or inhibition of episodes of AKI and/or chronically decreased perfusion may also be a treatment for Stage 4 or Stage 5 chronic kidney disease. Chronic kidney disease progression is described in National Kidney Foundation, K/DOQI Clinical Practice Guidelines for Chronic Kidney Disease: Evaluation, Classification and Stratification. Am. J. Kidney Dis. 39:S1-S266, 2002 (Suppl. 1), the disclosure of which is incorporated by reference herein in its entirety.

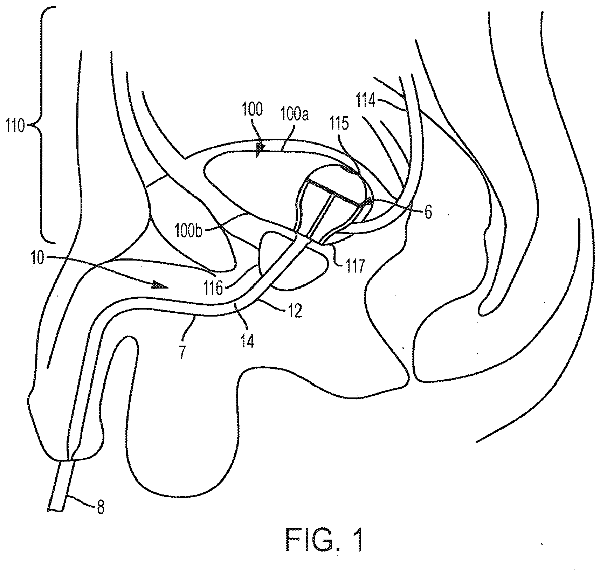

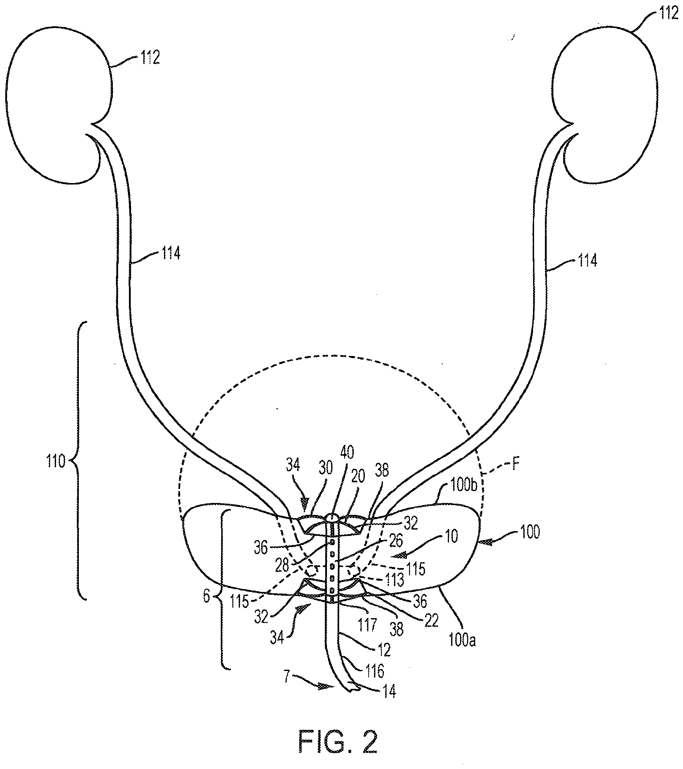

With reference to FIGS. 1 and 2, a urinary tract or urinary system 110 comprises a patient's right and left kidneys 112 (shown in FIG. 2). The kidneys 112 are responsible for blood filtration and clearance of waste compounds from the body through urine. Urine produced by the kidneys 112 is drained into a patient's bladder 100 through tubules referred to as ureters 114. For example, urine may be conducted through the ureters 114 by peristalsis of the ureter walls as well as by gravity. The ureters 114 enter the bladder 100 through a ureter orifice or opening 115. The bladder 100 is a flexible and substantially hollow structure adapted to collect urine until the urine is excreted from the body. The bladder 100 is transitionable from an empty position (shown in FIG. 2) to a full position (signified by reference line F in FIG. 2). Normally, when the bladder 100 reaches a substantially full state, urine is permitted to drain from the bladder 100 to a urethra 116 through a urethral sphincter or opening 117 located at a lower portion of the bladder 100. Contraction of the bladder 100 can be responsive to stresses and pressure exerted on the trigone region 113 of the bladder 100, which is the triangular region extending between the ureteral openings 115 and the urethral opening 117. The trigone region 113 is sensitive to stress and pressure, such that as the bladder 100 begins to fill, pressure on the trigone region 113 increases. When a threshold pressure on the trigone region is exceeded, sphincter or opening 117 relaxes and allows the bladder 100 to contract to expel collected urine through the urethra 116. The ureter orifice or openings 115 are covered by soft tissue which essentially forms a one-way flap valve. When the bladder 100 is collecting urine, the soft tissue is able to accommodate pressure from the peristalsis so that urine can pass from the ureters 114 into the bladder 100. When the bladder 100 contracts to expel urine therefrom, the soft tissue is restrained against the ureter openings 115 to prevent backflow of urine from the bladder 100 back into the ureters 114. The restraints are positioned to allow the ureter openings 115 to remain open during therapy so that negative pressure can draw urine into the bladder 100 and into catheter devices positioned in the bladder.

**Example catheter devices:**

With continued reference to FIGS. 1 and 2, exemplary disposable catheter devices 10 for inducing negative pressure in the bladder 100 of a patient are illustrated. As described herein, the bladder 100 is capable of contracting between the full position, denoted by dashed line F in FIG. 2, and the empty position. Desirably, the bladder 100 is prevented from collapsing completely by the catheter device 10. The exemplary catheter devices 10 can be a urine catheter (non-Foley) that can seal the bladder 100 and upper urinary system 110 under a guided negative pressure. The devices 10 generally comprise an elongated conduit or tubular body, referred to herein as a tube 12, having an external diameter or circumference which is generally within a range of about 8 to 16 Fr, the interior of which defines one or more drainage channel(s) or lumen(s) 14 or multiple lumens. The tube 12 can be formed from any suitable flexible material including, for example, biocompatible polymers, polyvinyl chloride, polytetrafluoroethylene (PTFE) such as Teflon®, silicon coated latex, or silicon. At least a portion or all of the catheter device 10, particularly the tube 12, can be coated with a hydrophilic coating to facilitate insertion and/or removal and/or to enhance comfort. In some examples, the coating is a hydrophobic and/or lubricious coating. For example, suitable coatings can comprise ComfortCoat® hydrophilic coating which is available from Koninklijke DSM N.V. or hydrophilic coatings comprising polyelectrolyte(s) such as are disclosed in United States Patent No. 8,512,795, which is incorporated herein by reference.

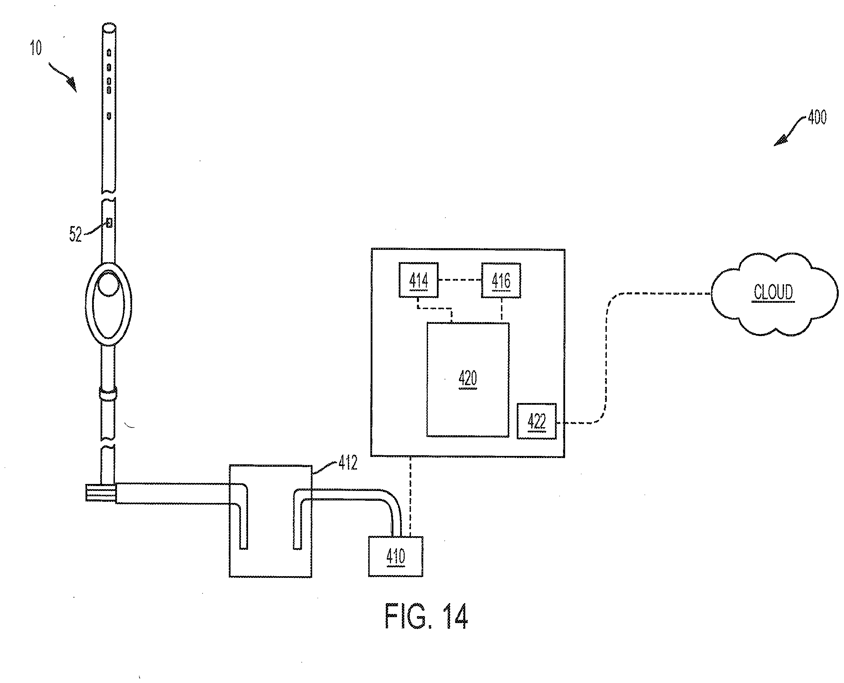

In some examples, the tube 12 comprises an indwelling portion 6 configured to be positioned in the bladder 100 and a second or middle indwelling portion 7 configured to extend through the urethra 116. Generally, the second portion 7 of the tube 12 is the same diameter as the indwelling portion 6 of the tube 12 described above, although the diameter may increase to facilitate flow. Alternatively, all or part of the second portion 7 of the tube 12 can be a separate tubing that is connected to the retention or indwelling portion 6 of the tube 12. The second portion 7 of the tube 12 does not include perforations, such as are described in the indwelling portion 6 of the tube 12, so as to prevent leakage from the side of the tube 12. The tube 12 further comprises an external portion 8 (shown in FIG. 1) which extends from the indwelling portions 6, 7 to an external fluid collection container or device, such as a pump 410 (shown in FIG. 14).

The catheter device 10 and, in particular, the tube 12, can be available in different lengths to accommodate anatomical differences for gender and/or patient size. For example, the average female urethra length is only a few inches, so the length of the tube 12 can be rather short. The average urethra length for males is longer due to the penis and can be variable. It is possible that woman can use catheter devices 10 with longer length tubes 12 provided that the excess tubing does not increase difficulty in manipulating or positioning the device 10. In some examples, the sterile portion of the catheter 10 can range from about 1 in to 3 inches, for women, to about 20 inches, for men. The total length of the tube 12 including sterile and non-sterile portions is about several feet.

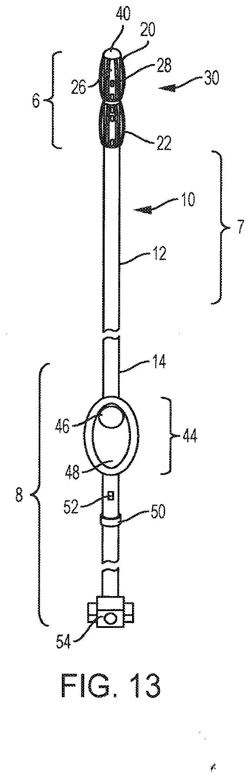

In some examples, the external portion 8 of the tube 12 comprises a deployment mechanism 44 and port 54 (shown in FIG. 13) for connection to the pump 410 (shown in FIG. 14). The connection between the tube 12 and the pump 410 or another fluid collection container can be a standard connection mechanism, such as a luer lock or snap fit connection. In other examples, a dedicated or customized connector or connection device can be used for connecting the proximal end of the catheter device 10 or port 54 to other elements of the fluid collection system. In some examples, the customized connector can be structured to prevent a user from connecting the catheter device 10 to unsuitable pressure sources. For example, the customized connector may be sized to prevent a user from connecting the catheter device 10 to sources of wall suction or other sources of elevated vacuum pressures.

*Exemplary single-stage fixation retention portions*

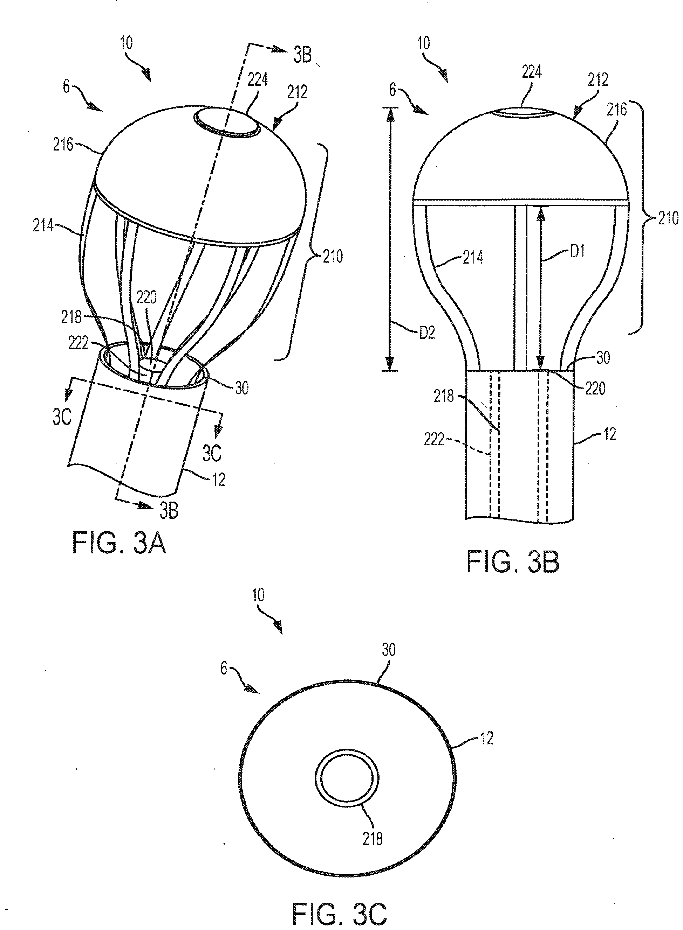

An exemplary indwelling portion 6 of a catheter device 10 is shown in FIGS. 3A-3C. The exemplary indwelling portion 6 of the catheter device 10 comprises a basket shaped structure, referred to as a bladder superior wall support 210 (shown in FIGS. 3A and 3B), configured to be disposed within a distal portion of the tube 12 in a retracted position and to extend from the distal end of the tube 12 in a deployed position. The bladder superior wall support 210 comprises a support cap 212 configured to support a superior wall 100a of the bladder 100 (shown in FIGS. 1 and 2) and a plurality of support members, such as legs 214, connected to a proximal surface of the support cap 212. The legs 214 can be positioned so that the cap 212 is spaced apart from an open distal end of the tube 12. For example, the legs 214 can be configured to maintain a gap, cavity, or space of distance D1 between an open distal end 30 of the tube 12 and the support cap 212. The distance D1 can be about 1.0 cm to about 2.0 cm. The height D2 of the bladder superior wall support 210 can about 1.5 cm to about 3.0 cm. The support cap 212 can be about 12 to 32 Fr in the deployed state, and preferably between about 8 mm and 10 mm.

In some examples, the legs 214 comprises flexible tines, which can be formed from a shape memory material, such as a nickel titanium. The support cap 212 can be a flexible cover 216 mounted to and supported by the legs 214. The flexible cover 216 can be formed from a soft and resilient material, such as silicone or Teflon®, for preventing air and/or fluid from passing through the cover 216. In some examples, the flexible material is formed from a material which does not appreciably abrade, irritate, or damage the mucosal lining of the bladder wall or the urethra when positioned adjacent to the mucosal lining, such as silicone or Teflon® materials. The thickness of the cover 216 can range from about 0.05 mm to about 0.5 mm. In some examples, the flexible cover 216 and legs 214 are sufficiently structurally rigid so that the cover 216 and legs 214 maintain their form when contacted by the superior wall 100a of the bladder 100 (shown in FIGS. 1 and 2). Accordingly, the legs 214 and flexible cover 216 prevent the bladder from collapsing and occluding perforations on the retention portion 6 and/or an open distal end 30 of the tube 12. In addition, the legs 214 and flexible cover 216 effectively keep the trigone region and ureteral orifices open so that negative pressure can draw urine into the bladder and lumen defined by or enclosed in the tube 12. As discussed herein, if the bladder were permitted to contract, flaps of tissue would extend over the ureter openings, thereby preventing negative pressure from drawing urine into the bladder.

In some examples, the support cap 212 is sized to be positioned within the bladder and to contact the superior wall of the bladder without occluding the ureteral openings. For example, the bladder superior wall support 210 may be appropriately sized to span the trigone region such that the trigone region and other portions of the bladder are restricted from contracting. By spanning and avoiding contact with the trigone region, the support cap 212 can be positioned away from the ureteral openings to prevent occlusion of the openings, which would inhibit or prevent urine flow from the ureters to the bladder.

In some examples, the catheter device 10 further comprises a drainage tube 218 defining a drainage lumen disposed at least partially within the tube 12. As shown in FIGS. 3A-3C, the drainage tube 218 can comprise an open distal end 220 positioned adjacent to or extending from the open distal end 30 of the tube 12. In some examples, the open distal end 220 of the drainage tube 218 is the only opening for drawing urine from the bladder into the interior of the drainage tube 218. In other examples, a distal portion of the drainage tube 218 may comprise perforations (not shown in FIGS. 3A-3C) or holes on a sidewall 222 thereon. The perforations can provide additional spaces for drawing urine into the interior of the drainage tube 218, thereby ensuring that fluid collection can continue even if the open distal end 220 of the drainage tube 218 is occluded. In addition, perforations can increase surface area available for drawing fluid into the drainage tube 218, thereby increasing efficiency and/or fluid collection yield.

In some examples, a distal most portion of the support cap 212 can comprise a sponge or pad 224, such as a gel pad. The pad 224 can be positioned to contact and press against the superior bladder wall 100a for the purpose of preventing drainage, aspiration, or other trauma to the bladder 100a during negative pressure treatment.

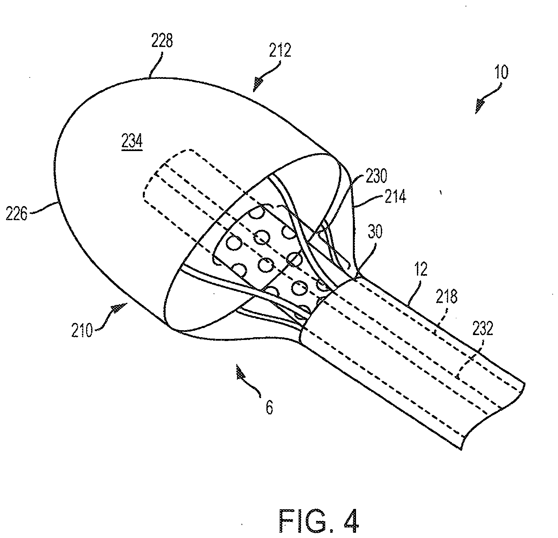

With reference to FIG. 4, an indwelling portion of another exemplary bladder catheter 10 including a bladder superior wall support 210 is illustrated. The bladder superior wall support 210 comprises a support cap 212 and a plurality of legs 214. As in previously described examples, the bladder superior wall support 210 is capable of being moved between a retracted position, in which the support 210 is at least partially retracted in a conduit or tube 12, and a deployed position to support the superior wall of the bladder. In some examples, the catheter device 10 also includes a drainage tube 218 extending from the open distal end 30 of the conduit or tube 12. Unlike in the previously-described examples, the support cap 212 shown in FIG. 4 comprises an inflatable balloon 226. The inflatable balloon 226 can be a substantially semi-spherical and can comprise a curved distal surface 228 configured to contact the superior bladder wall 100a when deployed.

In some examples, the drainage tube 218 comprises a perforated portion 230 extending between the open distal end 30 of the tube 12 and the support structure 212. The perforated portion 230 is positioned to draw fluid into an interior of the drainage tube 218 so that it can be removed from the bladder 100. Desirably, the perforated portion 230 is positioned so as not to be occluded either by the deployed support cap 212 or the bladder wall when negative pressure is applied thereto. The drainage tube 218 can comprise or be positioned adjacent to an inflation lumen 232 for providing fluid or gas to an interior 234 of the balloon 226 for inflating the balloon 226 from its contracted position to the deployed position. For example, as shown in FIG. 4, the inflation lumen 232 can be disposed within the drainage tube 218.

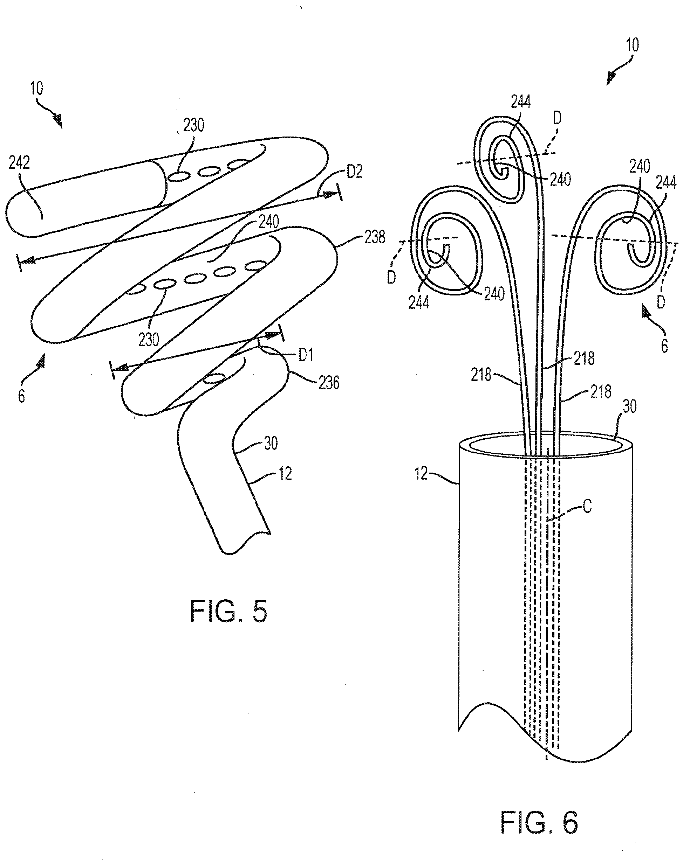

With reference to FIG. 5, an indwelling portion 6 of another exemplary urine collection catheter device 10 configured to be disposed in the patient's bladder is illustrated. The indwelling portion 6 comprises a coiled retention portion disposed at a distal end portion of the tube 12. In some examples, the retention portion 6 can be a separate structure that is connected to the distal end 30 of the tube 12. In other examples, coils can be imparted to the distal end portion of the tube 12, thereby forming the coiled retention portion 6. In some examples, the coiled retention portion 6 comprises at least a first coil 236 having a first diameter D1 and at least one second coil 238 having a second diameter D2. In some examples, the first diameter D1 is less than the second diameter D2, giving the retention portion 6 a tapered appearance. For example, the second diameter D2 can be about 4 mm to about 26 mm. The first diameter D1 can be about 2 mm to 13 mm. In other examples, the arrangement of coils in the coiled retention portion can be reversed, such that the distal-most coil has a larger diameter than one or more of the proximal coils.

The retention portion 6 of the tube 12 can further comprise a plurality of perforations 230 disposed on a radially inwardly facing side 240 of a sidewall of the retention portion 6. The diameter of the perforations 230 can range from about 0.005 mm to about 1.0 mm. The spacing between the perforations 230 can range from about 1.5 mm to about 15 mm. The perforations 230 can be spaced in any arrangement, for example, linear or offset. In some examples, the perforations 230 can be non-circular, and can have a surface area of about .00002 to 0.79 $mm^2$. Placing perforations 230 on the radially inwardly facing side 240 of the coiled retention portion 6 is intended to prevent the bladder from occluding the perforations 230 when negative pressure is applied through the catheter device 10. For example, in response to application of negative pressure to the bladder, portions of the bladder wall can be drawn against radially outwardly facing portions of the retention portion 6. Therefore, any perforations on radially outwardly facing side 242 of the retention portion 6 may be occluded by the bladder wall. However, perforations 230 on the radially inwardly facing side 240 of the retention portion 6 are protected. In other examples, a total surface area of perforations on the radially inwardly facing side 240 of the sidewall of the retention portion 6 can be greater than a total surface area of any perforations on radially outwardly facing side 242 of the retention portion 6.

With reference to FIG. 6, an exemplary retention portion 6 of a urine collection catheter device 10 including multiple coiled drainage lumens, generally denoted as lumens 218, is illustrated. The retention portion 6 comprises the tube 12 having a distal open end 30. The drainage lumens 218 are positioned partially within the tube 12. In a deployed position, the draining lumens 218 are configured to extend from the open distal end 30 of the tube 12 and to conform to a coiled orientation. The drainage lumens 218 can be separate for the entire length of the catheter device 10, or may empty into a single drainage lumen defined by the tube 12. In some examples, as shown in FIG. 6, the drainage lumens 218 can be pigtail coils having one or more coils 244. Unlike in the previously described example, the pigtail coils 244 are coiled about an axis that is not coextensive with an axis C of an uncoiled portion of the tube. Instead, as shown in FIG. 6, the pigtail coils can be coiled about an axis D that is approximately perpendicular to the axis C of the tube 12. In some examples, the drainage lumens 218 can comprise perforations (not shown in FIG. 6), similar to perforations 230 in FIG. 5, for drawing fluid from the bladder into an interior of the drainage lumens 218. In some examples, the perforations can be positioned on a radially inwardly facing side 240 of the coiled portions of the drainage lumens. As previously described, perforations positioned on radially inwardly facing sides of the drainage lumens 218 or tube 12 are less likely to be occluded by the bladder walls during application of negative pressure to the bladder. Urine can also be drawn directly into one or more drainage lumens defined by the tube 12. For example, rather than being drawing into the drainage lumen(s) 218 through the perforations 230, urine can be drawn directly through the open distal end 30 and into a drainage lumen defined by the tube 12.

*Exemplary retention portions with two-stage fixation*

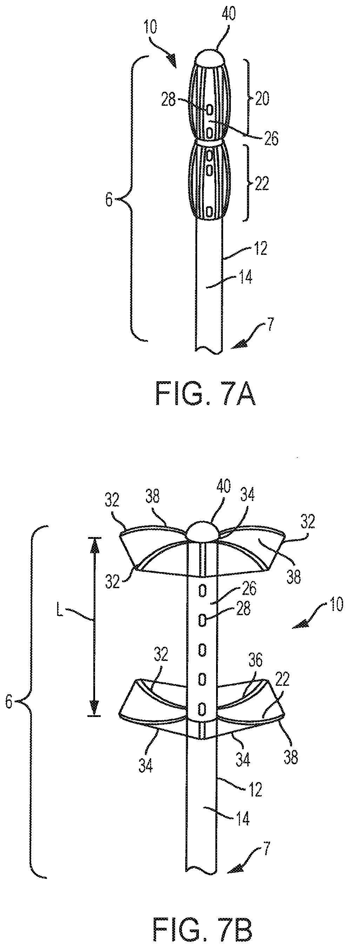

With reference to FIGS. 7A and 7B, an exemplary retention or indwelling portion 6, which is adapted to provide a two-stage passive temporary fixation within the bladder, is illustrated. The retention portion 6 is configured to be transitionable between a contracted position (as shown, for example, in FIG. 7A), during insertion to the bladder, and a deployed position (as shown in FIG. 7B) within the patient's bladder. As described herein, the indwelling portion 6 of the catheter device 10 comprises bracing, retaining, and/or sealing structures extending from the tube 12 for maintaining the bladder in a substantially expanded position through contact with the superior distal wall, in a similar manner to previously described examples. The two-stage fixation catheter device 10 also includes structures for sealing or partially sealing the urethral sphincter to prevent urine from passing through the urethra and for maintaining the trigone in an expanded position in which the ureteral orifices or openings are unobstructed.

In some examples, the indwelling portion 6 of the catheter device 10 comprises a bladder superior wall support, such as a distal anchor 20, and a bladder inferior wall support, such as a proximal anchor 22, each providing respective surfaces 20a, 22a for contacting the interior mucosal wall of the bladder. For example, the proximal anchor 22 can be positioned adjacent to the urethral sphincter to enhance suction when negative pressure is applied for drawing urine from the bladder. Desirably, the proximal anchor 22 is large enough to substantially or effectively seal the bladder and to stabilize the distal end 30 of the tube 12 within the bladder. For example, desirably, the proximal anchor 22 entirely seals the bladder with minimal leakage. It is noted that an 8 mm anchor is at least two times larger than the opening of the urethra. Accordingly, when correctly or substantially correctly positioned, the proximal anchor 22 covers the urethra opening with room to spare. In some examples, the proximal anchor 22 should not be so large that it completely covers the trigone and/or seals the ureter openings when positioned within the bladder. Furthermore, the distal anchor 20 also acts to maintain spacing between the superior bladder wall and the trigone so as to inhibit the superior bladder wall from contacting the trigone 113 (shown in FIG. 2).

While not intending to be bound by theory, it is believed that introducing negative pressure to the bladder 100 essentially collapses the bladder 100. Sealing the bladder 100 by positioning the proximal anchor 22 over the urethra opening may prevent the bladder 100 from collapsing completely, thereby ensuring that perforations or drainage holes 28 of the catheter 10, as well as the trigone and ureteral orifices or openings, are open, accessible, and free of obstruction. In some examples, pressure from the superior wall 100b holds the proximal anchor 22 in place against the inferior wall 100a, thereby creating the seal over the urethra opening.

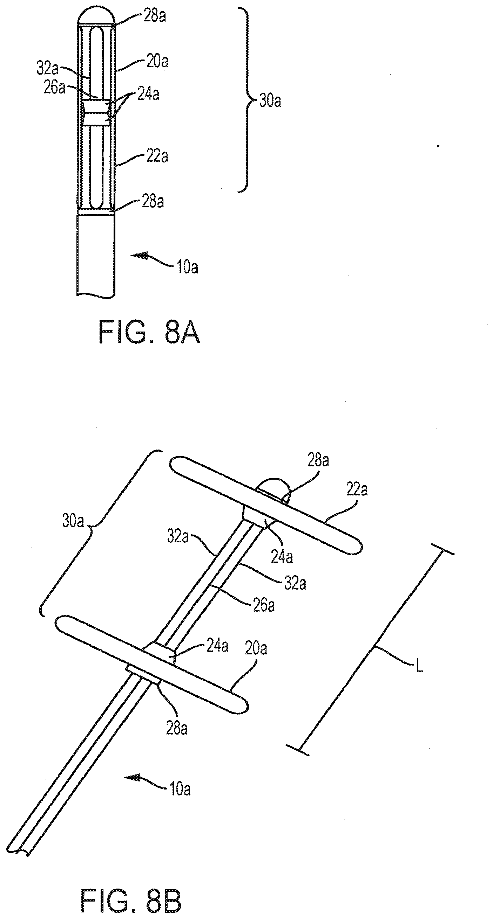

For a catheter device 10 including an 8 to 16 Fr elongated tube 12, the anchors 20, 22 can have a diameter equivalent to about 4 mm to 10.7 mm (12 to 32 Fr) in the deployed state, and preferably between about 8 mm and 10 mm (24 Fr and 30 Fr). It is believed that an 8 mm diameter anchor 20, 22 would be a single size suitable for all or most patients. For a catheter device 10 with 24 Fr anchors, the length L (shown in FIG. 7B) between the anchors 20, 22 is about 1.5 cm to about 2.3 cm, and preferably about 1.9 cm (0.75 in). Thus, in some examples, in the contracted state, the opposing anchors 20, 22 have a total non-overlapping length of about 1.6 cm (0.8 cm per anchor). To account for end padding and other spacing, the anchors 20, 22 can be separated by an additional small amount, such as an additional twenty percent (e.g. about 0.3 cm). Thus, the length L (shown in FIGS. 7B and 8B) between the anchors 20, 22 is preferably about 1.9 cm.

In some examples, the anchors 20, 22 are controlled by a release mechanism, such as a biasing member that, when actuated, causes the anchors 20, 22 to transition from the contracted position to the deployed position. When deployed, the anchors 20, 22 are positioned and configured to form an essentially or fully airtight seal with the bladder walls 100a, 100b and, in particular, to prevent air and/or urine from exiting the bladder 100 through the urethra 116. In some examples, when deployed, anchors 20, 22 of the indwelling portion 6 have sufficient integrity or rigidity to support the superior wall 100b of the bladder 100 and maintain a space between the superior wall and the trigone and/or inferior wall of the bladder. In some examples, when deployed, anchors 20, 22 substantially maintain their configuration upon deployment and do not collapse due to contact with bladder walls and/or trigone. In some examples, when deployed, the indwelling portion 6 maintains its orientation such that central axis thereof extends between the superior wall of the bladder and the trigone. Desirably, the indwelling portion 6 does not appreciably collapse along the central axis, or shift or tilt from its axial position upon deployment from pressure exerted on the indwelling portion 6 by the bladder walls.

In some examples, the distal end 30 of the catheter body or tube 12 extends through the distal anchor 20 and is in contact with the superior wall 100b of the bladder 100 (shown in FIGS. 1 and 2). In that case, the distal end 30 of the catheter body or tube 12 can include a sponge or pad 40, such as a gel pad, mounted to the catheter body or tube 12 and positioned to contact and press against the superior bl adder wall 100b for the purpose of preventing drainage, aspiration, or other trauma.

The tube 12 can further comprise a fluid receiving portion, e.g., the drainage channel(s) or lumen(s) 14. The drainage lumen 14 can include one or more perforations, such as drainage ports, eyelets, or holes 28 for draining fluid (e.g. urine or air) from the bladder 100 into the lumen 14 of the tube 12 for removal from the bladder 100. The drainage holes 28 can be arranged in any suitable pattern, such as linearly along the length of the catheter body or tube 12, at various positions around the tube 12 or in a helical pattern extending along the tube 12. Desirably, the drainage holes 28 are arranged in a pattern that ensures stability and rigidity of the distal end 30 in the deployed position. In some examples, the drainage lumen 14 comprises about one to twenty drainage holes 28. The drainage holes 28 can have a diameter of about 0.005 mm to about 0.5 mm. The holes 28 can be generally circular or oval shaped, and can be arranged in a straight line along the tube 12 or can be offset.

In some examples, as shown in FIGS. 7A and 7B, the anchors 20, 22 can comprise a basket or umbrella shaped frame structure. For example, the anchors 20, 22 can comprise flexible supports, such as flexible support members 32, extending radially from the tube 12. For example, each flexible member 32 can include one end that is fixedly connected to the tube 12 and a free end that, in the deployed position, extends radially outward from the tube 12. The flexible members 32 can be slightly bowed, curved, or biased to better absorb contracting forces from the superior bladder wall. The flexible members 32 can be any suitable length and width to correspond with the anatomy of the bladder 100. For example, the flexible members 32 can be formed from a shape-memory alloy, such as nickel titanium, can be generally cylindrical or columnar in shape, and can have a diameter of between about 0.05 mm and 1 mm. Tn some examples, the flexible members 32 can also include one or more hinges for imparting sufficient flexibility thereto.

The support members 32 can be covered by a support cap, such as a cover 38 or membrane, formed from a flexible and non-porous material or fabric, such as silicone or Teflon®, for preventing air and/or fluid from passing through the cover or membrane. In some examples, the cover 38 is formed from a material which does not appreciably abrade, irritate, or damage the mucosal lining of the bladder wall or the urethra when positioned adjacent to the mucosal lining, such as silicone or Teflon® materials. The thickness of the cover 38 can range from about 0.05 mm to about 0.5 mm. In some examples, the cover 38 of the proximal anchor 22 defines an annular seal extending around a central opening 34 thereof for sealing the inferior bladder wall. In some examples, portions of the cover 38 can comprise include an elastomeric material or other flexible material, such as silicone or Teflon®, for ensuring tight contact with the inferior bladder wall.

In some examples, the catheter device 10 can further comprise one or more posts 26 extending substantially parallel and/or concentric to the tube 12 between the proximal anchor 22 and the distal anchor 20 for providing additional support for the tube 12. In some examples, the posts 26 can be substantially rigid members that are connected to, enclosed within, or integrally formed with other portions of the tube 12. For example, the posts 26 can include one or more nickel titanium or plastic tines. The posts 26 are provided so that the distal end 30 of the tube 12 can withstand forces exerted on the anchors 20, 22 by the bladder wall(s). For example, the posts 26 can be formed from a rigid material that does not bend from a central axis of the tube 12 and ensures even distribution of downward bladder wall pressure. The length L (shown in FIG. 7B) of the post 26 can be a fixed length, and is selected such that, when deployed in the bladder 100, the anchors 20, 22 and the post 26 create sufficient space so that drainage functions of the catheter 10 are not inhibited. For example, the length L of the post 26 can be between about 1.0 cm and 3.0 cm. The posts 26 can prevent the bladder 100 from collapsing and obstructing drainage holes or anatomical structures of the bladder 100, such as the ureters 114. For anchors 20, 22 having a deployed diameter of about 8 mm, the posts 26 can have a diameter of about 0.3 mm to about 1.0 mm (e.g., 1 Fr to 3.3 Fr).

Alternatively, in some examples, the fluid receiving portion or distal end 30 of the tube 12 can be provided in different lengths L for different patients. In other arrangements, the distal end 30 can include a length adjustment mechanism, such as a telescoping arrangement, for adjusting the length L of the distal end 30 for a particular patient.

With reference to FIGS. 8A and 8B, an indwelling portion of another exemplary catheter device 10a is illustrated. A fluid receiving portion or distal end portion 30a of the catheter device 10a is shown in a contracted position in FIG. 8A, and in a deployed position in FIG. 8B. The distal end 30a includes opposing bladder wall supports for supporting the superior and inferior bladder walls. For example, the distal end portion 30a can comprise a proximal sheath 20a and a distal sheath 22a. Each sheath 20a, 22a extends between a slidable ring or collar 24a and stationary or mounted ring or collar 28a. The sheaths 20a, 22a are formed from a flexible, non-porous material, such as silicon. The sheaths 20a, 22a are held together by one or more flexible wires or cables 26a. The sheaths 20a, 22a can also be connected by one or more rigid members, such as supports 32a. In some examples, the supports 32a can be tines formed from a flexible, shape-memory material, such as nickel titanium. The supports 32a are positioned to provide support for the proximal sheath 20a and to prevent the distal end 30a from collapsing when it is in the deployed position. In the contracted position, the collars 24a, 28a are positioned apart from one another, such that the sheaths 20a, 22a are stretched or folded against the cable 26a and supports 32a. In the deployed position, the slidable collars 24a are moved toward the stationary collars 28a, allowing the sheaths 20a, 22a to unfold from the central cables 26a and to form a substantially flat disk-shaped structure.

In use, the distal end 30a of the catheter device 10a is inserted into the bladder of a patient in the contracted position. Once inserted in the bladder, the distal sheath 22a is released by sliding the slidable collar 24a in a distal direction toward the stationary collar 28a. Once the distal sheath 22a is deployed, the proximal sheath 20a is released or deployed in a similar manner by sliding the slidable collar 24a in the proximal direction toward the respective stationary collar 28a. At this point, the proximal sheath 20a is floating within the bladder, and is not positioned or sealed against the inferior wall of the bladder. Pressure against the distal sheath 22a caused by collapsing of the bladder is transferred to the proximal sheath 20a through the supports 32a and causes the proximal sheath 20a to move toward the desired position adjacent to the opening of the urethra. Once the proximal sheath 20a is in place, a seal over the urethra opening is created. The seal causes a negative pressure within the bladder and prevents air and/or urine from exiting the bladder through the urethra.

*Exemplary retention portions with annular inflatable balloons*

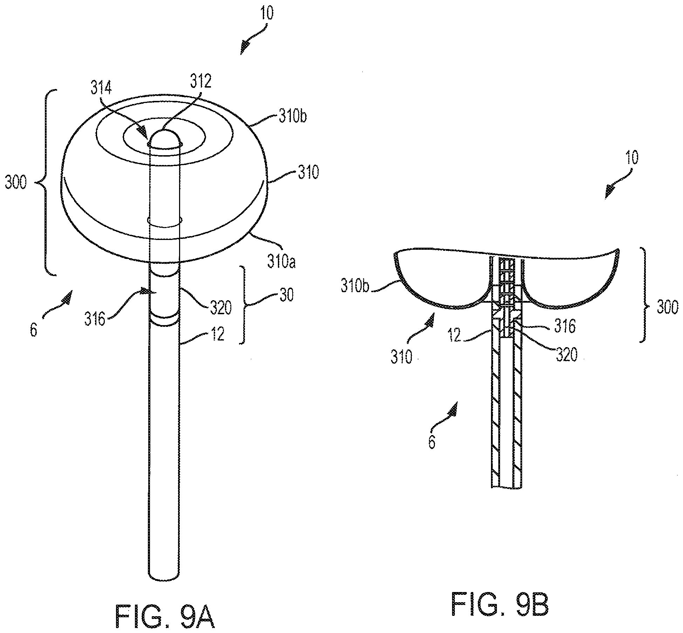

With reference to FIGS. 9A-11B, indwelling or retention portions 6 of additional exemplary urine collection catheter devices 10 comprising bladder superior wall supports 300 are illustrated. Unlike in previously described examples, the rigid or substantially rigid anchors 20, 22 (shown in FIGS. 7A and 7B) are replaced with an inflatable support cap, such as an annular balloon 310, positioned to contact the superior wall of the bladder to prevent the bladder from contracting and occluding either fluid port(s) 312 of the catheter device 10 or the ureteral openings of the bladder. In some examples, a distal end portion 30 of the tube 12 extends through a central opening 314 of the balloon 310. The distal end portion 30 of the tube 12 can also contact the superior bladder wall.

With specific reference to FIGS. 9A and 9B, in some examples, the tube 12 comprises a fluid access portion 316 positioned proximal to the balloon 310 and extending through a sidewall of the tube 12. The fluid access portion 316 can comprise a filter 318 (shown in FIG. 9B) disposed about a central lumen of the tube 12. In some examples, a sponge material 320 can be positioned over the filter 318 for increased absorbance of fluid within the bladder. For example, the sponge material 320 can be injection molded over the filter 318. In use, urine is absorbed by the sponge material 320 and, upon application of negative pressure through the tube 12, passes through the filter 318 and into the central lumen of the tube 12.

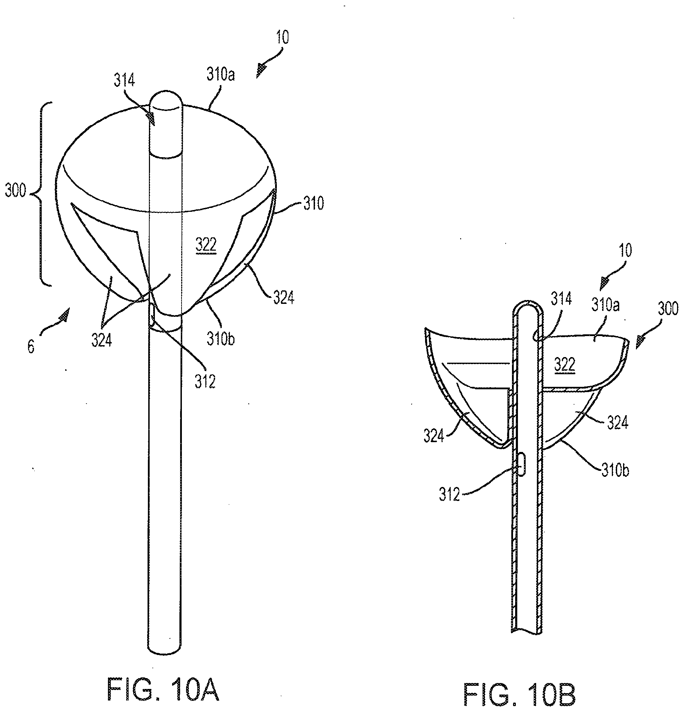

With specific reference to FIGS. 10A and 10B, in another exemplary embodiment, the support cap, such as the annular balloon 310, comprises a substantially bulbous distal portion 322 configured to contact and retain the superior bladder wall. The balloon 310 further comprises a plurality of proximally extending lobes 324. For example, the balloon 310 can comprise three lobes 324 spaced equidistantly around a portion of the tube 12 proximal to the balloon 310. As shown in FIG. 10B, the fluid ports 312 can be positioned between adjacent lobes 324. In this configuration, the lobes 324 and bulbous distal portion 322 contact the bladder wall, which prevents the bladder wall from blocking or occluding the fluid ports 312.

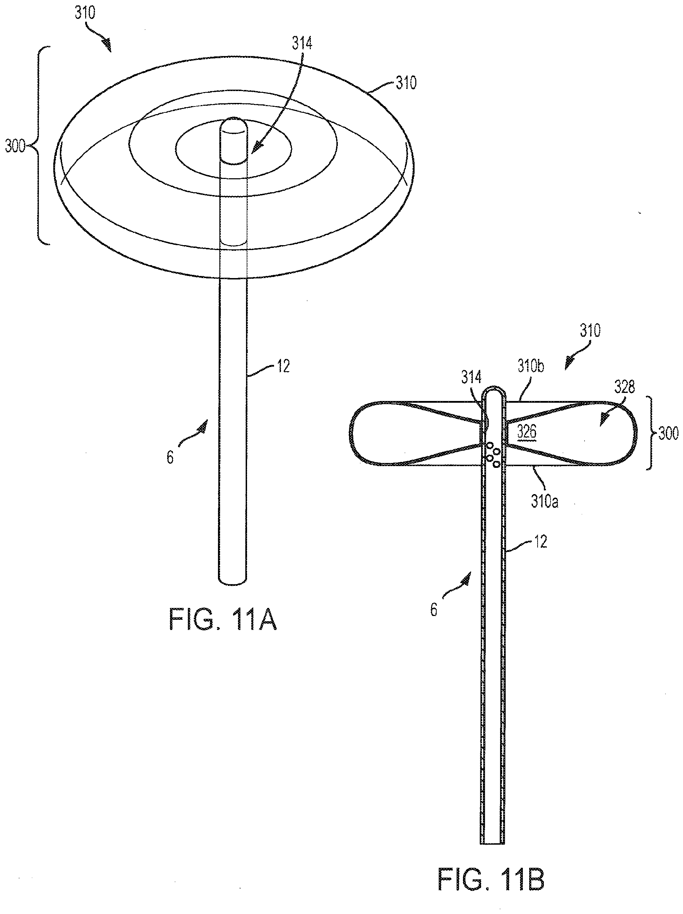

With specific reference to FIGS. 11A and 11B, in another exemplary embodiment, the annular balloon 310 is provided with a flattened and elongated shape. For example, the annular balloon 310 can have a substantially teardrop shaped radial cross section as shown in FIG. 11B, with a narrower portion 326 thereof positioned adjacent to the tube 12 and the enlarged or bulbous portion 328 positioned on the radially outwardly facing side thereof. The flatted annular balloon 310 is configured to span and seal the trigone region of the bladder such that when deployed in the bladder, the outer circumference of the balloon 310 extends radially beyond the ureteral openings. For example, when positioned in the patient's bladder, the central opening 314 of the balloon 310 can be configured to be positioned above the trigone region. Fluid port(s) 312 can be positioned proximal to the central portion balloon 310, as shown in FIG. 11B. Desirably, the fluid port(s) 312 are positioned between the central opening 314 of the balloon and the trigone region. When the bladder contracts from application of negative pressure, the bladder wall is supported by the outer circumference of the balloon 310 to avoid blocking the ureter openings. Accordingly, in this configuration, the balloon 310 contacts and prevents the bladder wall from blocking or occluding the fluid ports 312. In a similar manner, as discussed herein, the balloon 310 keeps the trigone region open so that urine can be drawn from the ureters into the bladder through the ureteral openings.

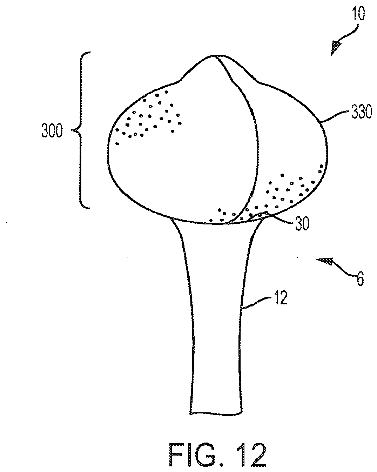

With reference to FIG. 12, another exemplary embodiment of an indwelling portion 6 of a urine collection catheter 10 comprising a bladder superior wall support 300 is illustrated. The bladder superior wall support 300 comprises a bulbous sponge 330 mounted to and extending from the open distal end 30 of the tube 12. The sponge 330 is substantially similar to the balloon 310 in previously-described examples and can be configured to contact the superior bladder wall to prevent or counteract contraction of the bladder. Desirably, the sponge 330 is formed from a soft, pliable material that does not appreciably abrade, irritate, or damage a mucosal lining of the bladder walls or of a urethra when positioned adjacent to a mucosal lining of the bladder walls or the urethra. In use, upon application of negative pressure through the tube 12, fluid is drawn through the porous sponge 330, through the distal opening 30, and into a central lumen of the tube 12.

*Exemplary external portions of the urine collection catheter*

With reference to FIG. 13, elements of the external portion 8 of the tube 12 will be described in detail. As previously discussed, the external portion 8 comprises portions of the tube 12 external to the patient's body. A proximal end of the external portion 8 can be configured to be connected to a fluid container or pump 410 (shown in FIG. 14). In some examples, the external portion 8 can comprise a deployment guide 44 for advancing or inserting the catheter 10 through the urethra 116 (shown in FIGS. 1 and 2) and into the bladder. Desirably, the deployment guide 44 is easy to use for non-medical personnel so that, for example, the device 10 can be deployed independently by the patient (e.g., self-administration). The deployment guide 44 can be configured to accommodate different catheter lengths to accommodate the anatomy of a particular patient. The deployment guide 44 can include a mechanism for advancing the catheter 10 such as a trigger 46 or advancing knob. In some examples, the user extends or lengthens the catheter device 10 by pressing the trigger 46 in a distal direction. Similarly, the catheter device 10 is retracted by pulling the trigger 46 in the opposite direction to withdrawn the catheter 10 from the patient.

The deployment guide 44 can further comprise a release mechanism 48 for releasing the anchors 20, 22 to transition the catheter 10 from the contracted position (shown in FIGS. 7A and 8A) to the deployed position (shown, for example, in FIGS. 7B and 8B). For example, the release mechanism 48 can comprise a release button or switch located on or adjacent to the deployment guide 44 that, when actuated by a user, causes a corresponding structure on the distal end 30 of the catheter 10 to push or move the anchors 20, 22 from the contracted position to the deployed position. More specifically, the anchors 20, 22 naturally remain in the contracted position. The release mechanism 48 overcomes the bias toward the contracted position and temporarily holds or locks the anchors 20, 22 in the deployed position. In some examples, the release mechanism 48 can be configured to release the anchors 20, 22 simultaneously to avoid applying unequal pressure to opposite sides of the bladder 100, as would occur if one anchor was in a deployed state while the other anchor was in a contracted state. Similarly, the release mechanism 48 can be configured to automatically retract the proximal anchor 22 if the distal anchor 20 is forced to close from an excessive force exerted by the bladder wall 100a, 100b. The release mechanism 48 can also be configured to automatically retract the anchors 20, 22 if, for example, the catheter device 10 is inadvertently or intentionally pulled out while the distal end 30 is in its deployed position.

With continued reference to FIG. 13, since the anchors 20, 22 are passively fixed and create an essentially airtight seal in the bladder 100 (shown in FIGS. 1 and 2), there is a risk that trauma to the bladder 100 or urethra 116 can occur if the catheter device 10 were pulled while the anchors 20, 22 were in the deployed state. Therefore, the external portion of the catheter 10 can comprise a breakaway valve 50 for releasing or separating a portion of the catheter 10 including the indwelling portion 6 of the catheter device 10 from the external portion of the catheter 10. The breakaway valve 50 is generally positioned along the tube 12 at a position that is proximal to the deployment guide 44 as shown, for example, in FIG. 13. The breakaway valve 50 can be configured such that, when the portions of the catheter device 10 are separated, the valve 50 transitions to a closed position to prevent fluid from leaking from the catheter 10. The external portions of the catheter 10 can be reattached to the breakaway valve 50 to continue therapy.

The catheter device 10 can further comprise sensors 52 for monitoring fluid characteristics of urine being excreted from the bladder. Information obtained from the sensors 52 can be transmitted to a central data collection module or processor and used, for example, to control operation of an external device, such as the pump 410 (shown in FIG. 14). The sensors 52 can be integrally formed with the tube 12 such as, for example, embedded in a wall of the tube 12 and in fluid communication with the one or more lumens 14 of the catheter 10. In other examples, one or more of the sensors 52 can be positioned in a fluid collection container (not shown) or in internal circuitry of an external device, such as a pump.

In some examples, the catheter device 10 further comprises one or more of the following types of sensors 52. For example, the catheter can include a conductance sensor or electrode that samples conductivity of urine in the catheter 10. The normal conductance of human urine is about 5-10 mS/m. Urine having a conductance outside of the expected range can indicate that the patient is experiencing a physiological problem, which requires further treatment or analysis. The catheter 10 can also include a flow meter for measuring a flow rate of urine through the catheter 10. Flow rate can be used to determine a total volume of fluid excreted from the body. The catheter 10 can also include a thermometer for measuring urine temperature. Urine temperature can be used to collaborate the conductance sensor. Urine temperature can also be used for monitoring purposes, as urine temperature outside of a physiologically normal range can be indicative of certain physiological conditions.

With continued reference to FIG. 13, the proximal end of the catheter body or tube 12 can comprise a port 54 configured to connect either to flexible tubing of a fluid collection system or directly to an external unit, such as a pump. The port 54 can comprise connectors, sealing members, and valves for forming a suitable fluid connection between the catheter 10 and the external unit. For example, the connector can be a bracket, luer connector, screw connector, clamp, or other suitable connection mechanism as is known in the art. In another example, the catheter body or tube 12 can be connected to a peristaltic pump connector. The peristaltic pump connector can be an inline catheter that is fed through arms of the pump, which are positioned to force fluid through the inline catheter. In other examples, the pump can be a diaphragm or piston pump, as are known in the art.

**Exemplary system for inducing negative pressure**

With reference to FIG. 14, a system 400 for inducing negative pressure including a catheter device 10 deployed in the bladder of a patient is illustrated. In some examples, the system 400 comprises the catheter device 10 connected to a fluid collection container 412 for collecting and storing expelled urine. The fluid collection container 412 can be placed under negative pressure by an external unit, such as the pump 410, connected thereto. The negative pressure generated by the pump 410 is provided to the patient's bladder through one or more drainage lumens of the catheter device 10. As discussed herein, negative pressure therapy can be provided for overcoming interstitial pressure in the kidneys to induce urine production. In some examples, the system 400 further comprises a controller 414, such as a microprocessor, having or associated with computer readable memory 416. The memory 416 can store instructions that, when executed, cause the controller 414 to receive information from sensors 52 located on, or associated with, the catheter device 10, determine information about the condition of the patient based on information from the sensors 52, and determine and implement operating parameters for the pump 410 based on information received from the sensors 52.

In some examples, the controller 414 is incorporated in a separate electronic device, such as a dedicated electronic device or a multipurpose electronic device, such as a computer, tablet PC, or smart phone. Alternatively, the controller 414 can be integral with and/or electronically coupled to the pump 410 and, for example, can control both a user interface for manually operating the pump 410, as well as system functions, such as receiving and processing information from the sensors 52.

The controller 414 can be configured to receive information from the one or more sensors 52, such as the conductance sensor, and to store the information in the associated computer-readable memory 416. For example, the controller 414 can be configured to receive information from the conductance sensor at a predetermined rate, such as once every second, and to determine a conductance based on the received information. In some examples, the algorithm for calculating conductance can also include other sensor measurements, such as urine temperature, to obtain a more robust determination of conductance.

The controller 414 can also be configured to calculate patient physical statistics or diagnostic indicators that illustrate changes in patient condition over time. For example, the system 400 can be configured to identify an amount of total sodium excreted. The total sodium excreted could be based, for example, on a combination of flow rate and conductance over a period of time.

With continued reference to FIG. 14, the system 400 can further comprise a feedback device 420, such as a visual display or audio system, for providing information to the user. In some examples, the feedback device 420 can be integrally formed with the pump 410. Alternatively, the feedback device 420 can be a separate dedicated or a multipurpose electronic device, such as a computer, laptop computer, tablet PC, smart phone, or other handheld electronic device. The feedback device 420 can be configured to receive the calculated or determined measurements from the controller 414 and to present the received information to a user via the feedback device 420. For example, the feedback device 420 can be configured to display current negative pressure (in mmHg) being applied to the urinary tract. The feedback device 420 can also display current flow rate of urine, temperature, current conductance in mS/m of urine, total urine produced during the session, total sodium excreted during the session, or any combination thereof.

The feedback device 420 can also include a user interface that allows the user to control operation of the pump 410. For example, the user can engage or turn off the pump 410 via the user interface. The user can also adjust pressure applied by the pump 410 to achieve a greater magnitude or rate of sodium excretion and fluid removal.

In some examples, the feedback device 420 and/or pump 410 further comprise a data transmitter 422 for sending information from the device 420 and/or pump 410 to other electronic devices or computer networks. The data transmitter 422 can utilize a short-range or long-range data communications protocol. An example of a short-range data transmission protocol is Bluetooth®. Long-range data transmission networks include, for example, Wi-Fi, Zigbee, cellular transmissions protocols, and the like. The data transmitter 422 can send information to a patient's physician or caregiver to inform the physician or caregiver about the patient's current condition. Alternatively, or in addition, information can be sent from the data transmitter 422 to existing databases or information storage locations, such as, for example, to include the recorded information in a patient's electronic health record (EHR).

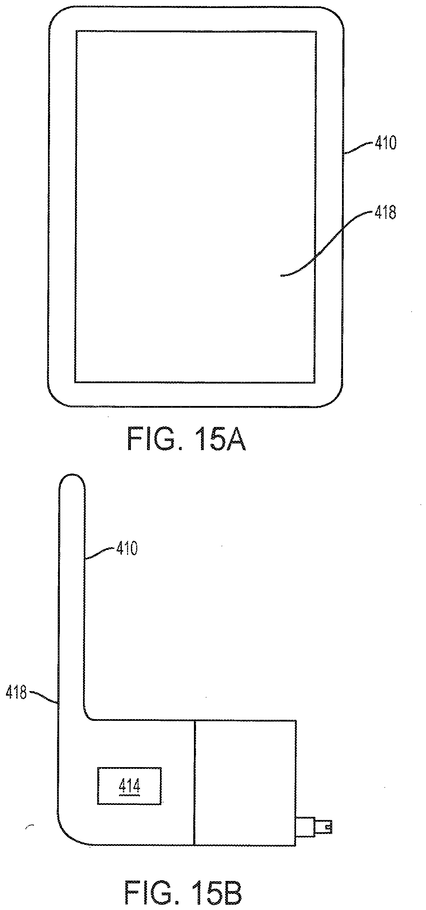

With reference to FIGS. 15A and 15B, an exemplary pump 410 for use with the system is illustrated. In some examples, the pump 410 is a micro-pump configured to draw fluid from the catheter device and having a sensitivity of about 0.5 mmHg. Desirably, the pump 410 is capable of providing a range of flow of urine between 0.05 ml/min and 3 ml/min for extended periods of time. At 0.2 ml/min it is anticipated that about 300 mL of urine per day is collected by the system 400. The pump 410 can be configured to provide a negative pressure to the bladder of the patient, the negative pressure ranging between about 0. 1 mmHg and 20 mmHg (gauge pressure at the pump 410). For example, a micro-pump manufactured by Langer Inc. (Model BT100-2J), can be used with the presently disclosed system 400. Diaphragm aspirator pumps, as well as other types of commercially available pumps, can also be used for this purpose. Peristaltic pumps can also be used with the system 400.

In some examples, the pump 410 can be configured for extended use and, thus, is capable of maintaining precise suction for periods of between 8 and 24 hours. The pump 410 can be manually operated and, in that case, includes a control panel 418 that allows a user to set a desired suction value. The pump 410 can also include a controller or processor, which can be the same controller that operates the system 400, or can be a separate processor dedicated for operation of the pump 410. In either case, the processor is configured for both receiving instructions for manual operation of the pump and for automatically operating the pump 410 according to predetermined operating parameters. Alternatively, or in addition, operation of the pump 410 can be controlled by the processor based on feedback received from the plurality of sensors associated with the catheter.

## Method of inducing negative pressure

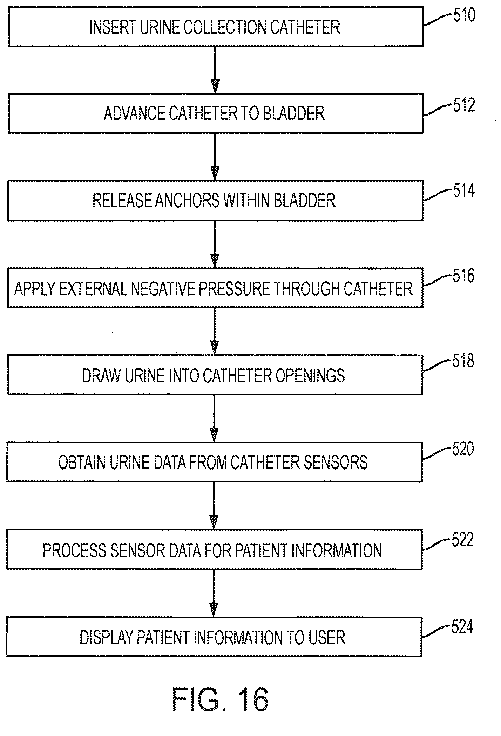

Having described the catheter device and system, a process for inducing negative pressure in the bladder will now be discussed in detail. With reference to FIG. 16, a medical professional, caregiver, or the patient inserts the catheter through the urethra of the patient, as shown in box 510. The catheter is advanced through the urethra and enters the bladder at box 512. The user advances the catheter through the bladder until the pad or padding on the distal tip of the catheter comes into contact with the bladder wall immediately adjacent to the urethral sphincter. At box 514, the user engages the release mechanism such that the proximal anchor and the distal anchor extend from the contracted state to the deployed state. In the deployed state, the distal anchor contacts the bladder wall immediately adjacent to the urethral sphincter. The proximal anchor contacts an opposing side of the bladder wall from the distal anchor and seals the opening to the urethra to maintain urine in the bladder. The anchors can be released simultaneously. In some examples, the distal anchor and the proximal anchor are also positioned simultaneously. Alternatively, the distal anchor can first be positioned adjacent to the urethral sphincter and the proximal anchor can float within the bladder. In that case, pressure from the superior wall of the bladder pushes against the distal anchor, which in turn guides the proximal anchor into position to seal the opening to the urethra.

Once the catheter is in place and transitioned to the deployed state, negative pressure is applied to the bladder at box 516. The negative pressure collapses the bladder, thereby holding the anchors against the mucosal wall to seal the urethra. Desirably, the negative pressure is evenly distributed across both ureters and both kidneys. Further, the negative pressure on the medulla counters congestion mediated interstitial hydrostatic pressures due to elevated intra-abdominal pressure and consequential or elevated renal venous pressure or renal lymphatic pressure. The applied negative pressure is therefore capable of increasing flow of filtrate through the medullary tubules and of decreasing water and sodium re-absorption.

As a result of the applied negative pressure, at box 518, urine is drawn into the catheter through the openings or eyelets. The urine is then drawn from the body through the catheter where it is collected in a collection container for disposal. As the urine is being drawn to the collection container, at box 520, the plurality of sensors provide a number of measurements about the urine that can be used to assess the volume of urine collected, as well as information about the physical condition of the patient and composition of the urine formed. For example, the sensors can be embedded in the catheter in fluid communication with the lumens extending therethrough. Information can be obtained by the sensors as urine passes through the catheter. The information obtained by the sensors can be processed, at box 522, by a processor associated with the pump or other device and, at box 524 is displayed to the user via the visual display of the feedback device.

The embodiments have been described with reference to various examples. Modifications and alterations will occur to others upon reading and understanding the foregoing examples. Accordingly, the foregoing examples are not to be construed as limiting the disclosure.

**Claims**

1. A bladder catheter, comprising:

   (a) a proximal portion; and
   (b) a distal portion, the distal portion comprising an inwardly facing side comprising one or more perforations and an outwardly facing side, and wherein, when negative pressure is applied through the bladder catheter, fluid is drawn into the bladder catheter through the one or more perforations while bladder walls are prevented from appreciably occluding the one or more perforations.

2. The bladder catheter of claim 1, wherein the distal portion comprises a retention portion which comprises the inwardly facing side.

3. The bladder catheter of claim 2, wherein the retention portion comprises a coiled retention portion extending radially within a patient's bladder.

4. The bladder catheter of claim 1 or 2, wherein the proximal portion extends outside a patient's body through the patient's urethra.

5. The bladder catheter of claim 1 or 2, wherein the proximal portion is configured to be connected to a pump for application of negative pressure through the bladder catheter.

EP 3 470 107 A1

FIG. 1

18

FIG. 2

FIG. 3A

FIG. 3B

FIG. 3C

FIG. 4

FIG. 5

FIG. 6

FIG. 7A

FIG. 7B

FIG. 8A

FIG. 8B

FIG. 9A

FIG. 9B

FIG. 10A

FIG. 10B

FIG. 11A

FIG. 11B

FIG. 12

FIG. 13

EP 3 470 107 A1

FIG. 14

410

418

## FIG. 15A

410

418

414

## FIG. 15B

INSERT URINE COLLECTION CATHETER — 510

↓

ADVANCE CATHETER TO BLADDER — 512

↓

RELEASE ANCHORS WITHIN BLADDER — 514

↓

APPLY EXTERNAL NEGATIVE PRESSURE THROUGH CATHETER — 516

↓

DRAW URINE INTO CATHETER OPENINGS — 518

↓

OBTAIN URINE DATA FROM CATHETER SENSORS — 520

↓

PROCESS SENSOR DATA FOR PATIENT INFORMATION — 522

↓

DISPLAY PATIENT INFORMATION TO USER — 524

FIG. 16

Europäisches
Patentamt

European
Patent Office

Office européen
des brevets

# EUROPEAN SEARCH REPORT

Application Number

EP 18 18 3523

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2003/181842 A1 (GELLMAN BARRY N [US]) 25 September 2003 (2003-09-25) * the whole document * | 1-5 | INV. A61M27/00 A61M1/00 A61F13/20 |
| Y | US 2004/193098 A1 (WENTLING ANGELA [US] ET AL) 30 September 2004 (2004-09-30) * paragraph [0057]; figures 2A-3 * | 1-5 | A61M25/04 A61M25/10 A61F13/15 |
| A | US 5 078 684 A (YASUDA KENICHI [JP]) 7 January 1992 (1992-01-07) * column 2, line 64 - line 68 * * figures 1,3 * | 1-4 | |

TECHNICAL FIELDS
SEARCHED     (IPC)

A61M

The present search report has been drawn up for all claims

| Place of search | Date of completion of the search | Examiner |
|---|---|---|
| Berlin | 7 March 2019 | Amaro, Henrique |

EPO FORM 1503 03.82 (P04C01)

## ANNEX TO THE EUROPEAN SEARCH REPORT
## ON EUROPEAN PATENT APPLICATION NO.

EP 18 18 3523

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2019

| Patent document cited in search report | | Publication date | Patent family member(s) | | Publication date |
|---|---|---|---|---|---|
| US 2003181842 | A1 | 25-09-2003 | AU 2003213828 A1<br>US 2003181842 A1<br>WO 03079934 A1 | | 08-10-2003<br>25-09-2003<br>02-10-2003 |
| US 2004193098 | A1 | 30-09-2004 | CA 2529414 A1<br>EP 1735028 A1<br>JP 4852533 B2<br>JP 2007532237 A<br>US 2004193098 A1<br>WO 2005107831 A1 | | 17-11-2005<br>27-12-2006<br>11-01-2012<br>15-11-2007<br>30-09-2004<br>17-11-2005 |
| US 5078684 | A | 07-01-1992 | DE 3886056 D1<br>DE 3886056 T2<br>EP 0418381 A1<br>JP H0510938 B2<br>JP S6480367 A<br>US 5078684 A<br>WO 8902281 A1 | | 13-01-1994<br>05-05-1994<br>27-03-1991<br>12-02-1993<br>27-03-1989<br>07-01-1992<br>23-03-1989 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62194585 B
- US 62260966 B
- US 62278721 B
- US 62300025 B
- US 8512795 B

### Non-patent literature cited in the description

- **JESSUP M.** The cardiorenal syndrome: Do we need a change of strategy or a change of tactics?. *JACC,* 2009, vol. 53 (7), 597-600
- **VERBRUGGE et al.** The kidney in congestive heart failure: Are natriuresis, sodium, and diruetucs really the good, the bad and the ugly?. *European Journal of Heart Failure,* 2014, vol. 16, 133-42
- **DAMMAN K.** Importance of venous congestion for worsening renal function in advanced decompensated heart failure. *JACC,* 2009, vol. 17, 589-96
- **PETERS, C.D.** Short and Long-Term Effects of the Angiotensin II Receptor Blocker Irbesartanon Intradialytic Central Hemodynamics: A Randomized Double-Blind Placebo-Controlled One-Year Intervention Trial (the SAFIR Study). *PLoS ONE,* 2015, vol. 10 (6), e0126882
- **BART B.** Ultrafiltration in decompensated heart failure with cardiorenal syndrome. *NEJM,* 2012, vol. 367, 2296-2304
- The Criteria Committee of the New York Heart Association. Nomenclature and Criteria for Diagnosis of Diseases of the Heart and Great Vessels. Little, Brown & Co, 1994, 253-256
- K/DOQI Clinical Practice Guidelines for Chronic Kidney Disease: Evaluation, Classification and Stratification. Am. J. Kidney Dis. National Kidney Foundation, 2002, vol. 39, S1-S266